# FASCICULE DE BREVET EUROPEEN

(11) **EP 2 229 408 B1**
(45) Date de publication et mention de la délivrance du brevet: **30.11.2011**
(21) Numéro de dépôt: 08872504.9
(22) Date de dépôt: 18.12.2008
(51) Int. Cl.: C07K 14/705, C07K 7/08, C12N 15/12, A61K 38/10, A61K 38/17, A61P 25/24

(54) **PEPTIDE DERIVE DU RECEPTEUR 3 DE LA NEUROTENSINE ET UTILISATION DANS LE TRAITEMENT DE MALADIES PSYCHIATRIQUES**
AUS DEM NEUROTENSIN-REZEPTOR 3 ENTWICKELTES PEPTID UND SEINE VERWENDUNG BEI DER BEHANDLUNG PSYCHISCHER KRANKHEITEN
PEPTIDE DERIVED FROM NEUROTENSIN RECEPTOR 3 AND USE THEREOF IN THE TREATMENT OF PSYCHIATRIC DISEASES

(30) Priorité: 21.12.2007 EP 07291602
(43) Date de publication de la demande: 22.09.2010
(73) Titulaire: Centre National de la Recherche Scientifique - CNRS, 75794 Paris (FR); Universite De Nice Sophia Antipolis, 06108 Nice Cedex 2 (FR)
(72) Inventeur: MAZELLA, Jean, F-06410 BIOT (FR); PETRAULT, Olivier, F-59160 Lomme (FR); BORSOTTO, Marc, F-06130 Grasse (FR); HEURTEAUX, Catherine, F-06600 Antibes (FR); WIDMANN, Catherine, F-06480 La Colle Sur Loup (FR)
(74) Mandataire: Novagraaf Technologies
(86) Numéro de dépôt international: PCT/FR2008/001784
(87) Numéro de publication internationale: WO 2009/103898

(56) Documents cités:
- EP-A1- 2 077 278
- WO-A-99/37762
- WO-A-2004/056385
- WESTERGAARD UFFE B ET AL: "Functional organization of the sortilin Vps10p domain" JOURNAL OF BIOLOGICAL CHEMISTRY, vol. 279, no. 48, 26 novembre 2004 (2004-11-26), pages 50221-50229, XP002478369 ISSN: 0021-9258
- DICOU ELENI ET AL: "Neurotensin receptor-3/sortilin mediates neurotensin-induced cytokine/chemokine expression in a murine microglial cell line" JOURNAL OF NEUROSCIENCE RESEARCH, vol. 78, no. 1, 1 octobre 2004 (2004-10-01), pages 92-99, XP002478370 ISSN: 0360-4012
- MAZELLA ET AL: "Functional roles of the NTS2 and NTS3 receptors" PEPTIDES, ELSEVIER, AMSTERDAM, US, vol. 27, no. 10, octobre 2006 (2006-10), pages 2469-2475, XP005661215 ISSN: 0196-9781
- ALLOUI ABDELKRIM ET AL: "TREK-1, a K+ channel involved in polymodal pain perception" EMBO (EUROPEAN MOLECULAR BIOLOGY ORGANIZATION) JOURNAL, vol. 25, no. 11, juin 2006 (2006-06), pages 2368-2376, XP002478371 ISSN: 0261-4189 cité dans la demande
- HEURTEAUX CATHERINE ET AL: "Deletion of the background potassium channel TREK-1 results in a depression-resistant phenotype" NATURE NEUROSCIENCE, vol. 9, no. 9, septembre 2006 (2006-09), pages 1134-1141, XP002478372 ISSN: 1097-6256
- HEURTEAUX C ET AL: "TREK-1, a K+ channel involved in neuroprotection and general anesthesia" EMBO (EUROPEAN MOLECULAR BIOLOGY ORGANIZATION) JOURNAL, vol. 23, no. 13, 7 juillet 2004 (2004-07-07), pages 2684-2695, XP002536216 ISSN: 0261-4189 cité dans la demande
- JANSEN PERNILLE ET AL: "Roles for the pro-neurotrophin receptor sortilin in neuronal development, aging and brain injury" NATURE NEUROSCIENCE, vol. 10, no. 11, novembre 2007 (2007-11), pages 1449-1457, XP002478373 ISSN: 1097-6256
- MAZELLA JEAN ET AL: "Spadin, a Sortilin-Derived Peptide, Targeting Rodent TREK-1 Channels: A New Concept in the Antidepressant Drug Design", PLOS BIOLOGY, vol. 8, no. 4, April 2010 (2010-04), page Article No.: e1000355, ISSN: 1544-9173(print)
- MAZELLA JEAN ET AL: "Spadin, a Sortilin-Derived Peptide, Targeting Rodent TREK-1 Channels: A New Concept in the Antidepressant Drug Design", PLOS BIOLOGY, vol. 8, no. 4, April 2010 (2010-04), page Article No.: e1000355, ISSN: 1544-9173(print)

## Description

### Domaine technique de l'invention

La présente invention se rapporte à un peptide dérivé du récepteur 3 de la neurotensine (NTSR3) et à son utilisation dans le traitement de maladies psychiatriques. La présente invention se rapporte en particulier à l'utilisation de ce peptide pour la fabrication d'un médicament, par exemple un antidépresseur.

La présente invention trouve une application dans les secteurs de l'industrie pharmaceutique et notamment dans les domaines de développement du médicament utilisé dans le traitement des maladies psychiatriques.

La présente invention trouve en particulier une application dans le développement d'un nouvel antidépresseur. Elle trouve également une application dans le traitement de la douleur.

Dans la description ci-dessous, les références entre parenthèses **(x)** renvoient à la liste des références à la fin des exemples.

### Art antérieur

Le document WO 2004/056385 décrit la séquence peptidique du propeptide de la neurotensine et décrit que ce dernier a une affinité pour le récepteur NTR3

L'article WESTERGAARD UFFE ET AL : « Functional organisation of the sortilin VPS10p domain » publié en novembre 2004 décrit la liaison d'un propeptide du récepteur NSTR3 avec le récepteur de la neurotensine NSTR3.

Les maladies psychiatriques représentent un réel problème de Santé Publique. Les travaux les plus récents ont confirmé la forte prévalence de la dépression : sur leur vie entière, 20% des femmes et 10% des hommes ont fait, font ou feront un épisode dépressif comme décrit dans Wong, M. & Licinio, J. «Research and treatment approaches to depression» Nat Rev Neurosci. 2, 343-351 (2001) **(1)**. De tels chiffres sont à l'évidence marquants; ils le sont plus encore lorsqu'on s'intéresse à la complication majeure de la dépression, le suicide, qui se chiffre à 12000 décès par an dans des pays comme la France comme décrit dans Moller HJ. «Suicide, suicidality and suicide prevention in affective disorders » Acta Psychiatr Scand 418 (suppl) : 73-80 (2003) **(2)**.

La dépression est une maladie très fréquente et souvent handicapante. Elle peut toucher jusqu'à 20% de la population dans les pays industrialisés. Ses origines sont diverses et multiples. Cette pathologie affecte aussi bien le psychisme que le comportement et la physiologie des patients. Les traitements de la dépression sont également multiples et les mécanismes d'action des médicaments utilisés ne sont pas clairement établis.

L'organisation mondiale de la santé (OMS) prévoit que la dépression unipolaire sera la deuxième cause de handicap en 2020. A la souffrance personnelle et familiale que représente la dépression s'ajoute le poids social important de cette pathologie. La dépression représente déjà l'une des premières causes d'arrêt du travail, avec une charge économique qui s'élève à plus de 30 milliards d'euros par an. Malgré l'arsenal thérapeutique mis à disposition du corps médical, en particulier les SSRI (*«selective serotonin reuptake inhibitors »*) et SNRI (*«serotonin norepinephrine reuptake inhibitors* »), 30% de la population dépressive n'a pas de traitement. Par ailleurs, le délai d'action des antidépresseurs est de l'ordre de 3 à 6 semaines et les effets secondaires sont souvent importants.

D'une façon générale, on estime que 15% des patients déprimés décèdent par suicide. Chez la plupart des malades, la dépression est due à l'interaction entre une prédisposition génétique et des facteurs environnementaux comme le stress ou les traumatismes émotionnels comme décrit dans Nestler E. Barrot M., DiLeone R. J.,Eisch A. J.,Gold S. J.,Monteggia, L. M. «Neurobiology of dépression » Neuron 34, 13-25 (2002) **(3).**

La maladie est fréquente et le marché des antidépresseurs (AD) est immense (au moins 10 milliards d'euros par an).

Néanmoins, si ces antidépresseurs améliorent l'état des patients dans environ 70% des cas, ils n'entraînent une rémission complète de la maladie que chez 30 à 40% d'entre eux. De plus, près d'un tiers des sujets traités résiste aux traitements existants. Cet état de fait oblige donc à envisager de nouveaux traitements, capables de prendre en compte les mécanismes de la dépression **(3)**.

Dans l'arsenal thérapeutique mis à disposition du corps médical, les antidépresseurs tricycliques (TCA) avec l'amitriptyline et l'imipramine ont été les premiers découverts, suivis par les inhibiteurs de la monoamine oxydase (IMAO), irréversibles et non sélectifs comme la phénelzine et la pargyline. Les effets indésirables, en particulier la cardiotoxicité des TCA (surtout en cas de surdosage) et les crises hypertensives des IMAO (interactions avec la tyramine alimentaire, le fameux « *cheese effect »*) ont poussé la recherche vers de nouvelles molécules d'efficacité thérapeutique identique mais de meilleure acceptabilité.

La notion de sélectivité est alors apparue avec les inhibiteurs spécifiques de la recapture de la noradrénaline (NA) ou de la sérotonine (5-hydroxytryptamine ou 5-HT). Les essais cliniques de phase III ont démontré pour ces nouvelles molécules une efficacité équivalente aux antidépresseurs de première génération et une meilleure sécurité, notamment en cas de surdosage.

Les inhibiteurs spécifiques de la recapture de la sérotonine (ISRS) et les inhibiteurs spécifiques de la recapture de la noradrénaline (ISRN) sont actuellement les molécules les plus utilisées comme décrit dans Baghai TC, Volz HP, Moller HJ. « Drug treatment of depression in the 2000s: An overview of achievements in the last 10 years and future possibilities » World J Biol Psychiatry; 7: 198-222 (2006) **(4)** et dans Weilburg JB. «An overview of SSRI and SNRI therapies for depression» Manag Care; 13 (6 Suppl Depression): 25-33 (2004) **(5)**. Les AD sont ainsi le plus souvent associés à une facilitation de la transmission des systèmes monoaminergiques.

Bien que la sérotonine, la noradrénaline et la dopamine sont impliquées de façon certaine, il est admis aujourd'hui que les modifications des taux de monoamines produites par les AD et les processus adaptatifs qui en découlent, en particulier l'altération de la sensibilité de certains de leurs récepteurs, ne peuvent expliquer à eux seuls le mécanisme d'action des antidépresseurs.

Ainsi, il est difficile de corréler le délai de 3 à 6 semaines nécessaire à l'obtention des AD avec l'augmentation des taux synaptiques de monoamines, qui intervient dès la première administration du produit. En près d'un demi-siècle, le nombre d'hypothèses sur la pathogénèse de la dépression et son traitement n'a cessé d'évoluer.

Par exemple, des concentrations élevées de glucocorticoïdes sont généralement associées à un effet négatif de l'humeur, ainsi que des altérations structurelles de l'hippocampe, par l'intermédiaire d'une diminution de la synthèse de BDNF (*« brain-derivated neurotrophic factor*»), d'une sécrétion excessive d'acide glutamique et/ou d'une diminution de la capture du glucose comme décrit dans Manji HK, Gottesman II, Gould TD. «Signal transduction and genes-to-behaviors pathways in psychiatric diseases« Sci STKE; 207 : pe49 (2003) **(6)**.

Conformément à ces observations, des inhibiteurs de la synthèse des glucocorticoïdes et des antagonistes des récepteurs de glucocorticoïdes exercent des effets de type AD comme décrit dans Reus VI, Wolkowitz OM. «Antiglucocorticoid drugs in the treatment of depression« Expert Opin Investig Drugs ; 10 : 1789-1796 (2001) **(7)**.

Des antagonistes agissant sur les récepteurs de la substance P, en particulier le NK1, ou le récepteur CRF (« *corticotropin-releasing factor»*)*,* ainsi que des antagonistes des récepteurs du NMDA ont été développés avec une certaine efficacité (voir Griebel G, Simiand J, Steinberg R, et al. «4-(2-Chloro-4-methoxy-5-methylphenyl)-N-[(1S)-2-cyclopropyl-1-(3-fluoro-4methylphenyl)ethyl]5-methyl-N-(2-propynyl)-1,3- thiazol-2-amine hydrochloride (SSR125543A), a potent and selective corticotrophin-releasing factor (1) receptor antagonist. Il. Characterization in rodent models of stress-related disorders » J Pharmacol Exp Ther ; 301 : 333-345 (2002) **(8)**; Kramer MS, Cutier N, Feighner J, et al. «Distinct mechanism for antidepressant activity by blockade of central substance P receptors » Science ; 281 : 1640-1645 (1998) **(9)** et Skolnick P. «Antidepressants for the new millennium » Eur J Pharmacol ; 375 : 31-40 (1999) **(10)**.

Diverses études récentes réalisées dans des situations de stress et des modèles de dépression ont impliqué la neurogenèse dans l'étiologie des troubles dépressifs majeurs comme décrit dans Kempermann G, Kronenberg G. «Depressed new neurons : Adult hippocampal neurogenesis and a cellular plasticity hypothesis of major depression« Biol Psychiatry, 54: 499-503 (2003) (11); Malberg JE, Schecter LE. Increasing hippocampal neurogenesis: a novel mechanism for antidepressant drugs » Curr Pharm Des; 11: 145-155 (2005) (12) et Duman, R. & Monteggia, L. «A neurotrophic model for stress-related mood disorders » Biol Psychiatry; 9 : 1116-1127 (2006) **(13).** Il a été démontré que tous les traitements chroniques AD, y compris l'électrochoc stimulent la prolifération des cellules progénitrices à l'origine des neurones de la couche granulaire de l'hippocampe.

On sait également que les AD modulent l'expression de différents facteurs impliqués dans la survie et la croissance des cellules, tels que la CREB, le Bcl2 ou les MAP-kinases. Toutefois, l'importance fonctionnelle de ces neurones néoformés dans la physiopathologie des troubles de l'humeur reste controversée (voir Henn FA, Vollmayr B. «Neurogenesis and depression: etiology or epiphenomenon? » Biol Psychiatry, 56 : 146-50 (2004) **(14)**).

L'ensemble de ces indications montrent que la dépression est une maladie complexe dont la physiopathologie est multifactorielle et, en conséquence, le traitement d'une telle pathologie reste un challenge.

Depuis plus de quarante ans, la recherche sur la dépression et le développement de médicaments efficaces ont été dominés par l'hypothèse monoaminergique. Bien que les neurotransmetteurs monoaminergiques (sérotonine, noradrénaline et dopamine) sont impliqués de façon indiscutable, le nombre d'hypothèses sur la physiopathologie de la dépression et les mécanismes d'action des AD n'a cessé d'évoluer.

Les effets secondaires connus des antidépresseurs sont liés aux modes d'action de ceux-ci. Par exemple, les antidépresseurs peuvent induire de la tachycardie, une prise de poids, une baisse de la libido, des accès sudatifs, une baisse de l'appétit et des altérations neurologiques, telles que des céphalées, des accidents vasculaires cérébraux (AVC) et des crises d'épilepsie.

Les médicaments AD utilisés aujourd'hui produisent une gamme d'effets indésirables, parmi lesquels la sécheresse de la bouche, la vision floue et l'altération de la fonction intestinale (diarrhée ou constipation)._Même si beaucoup d'effets secondaires sont passagers (comme la nausée), certains semblent être constants au fil du temps (comme les effets sexuels) et risquent d'affecter l'assiduité au traitement à long terme. C'est la raison pour laquelle la recherche de nouvelles molécules agissant sur des récepteurs ou canaux nouvellement identifiés dans la dépression est cruciale.

Certaines protéines (récepteurs et canaux) ont été impliquées dans les mécanismes moléculaires de la dépression. C'est notamment le cas du récepteur 3 de la neurotensine (NTSR3), originellement appelé sortiline et du canal potassique de fond TREK-1 dont l'inactivation chez la souris produit un phénotype de résistance à la dépression (Heurteaux, Lucas, Guy, El Yacoubi, Thümmler, Peng, Noble, Blondeau, Widmann et al. «Deletion of TREK-1, a background potassium channel, results in a dépression-résistant phénotype » Nature Neurosci.,9, 1134-1141 (2006) **(15)**). Aucune molécule interagissant efficacement avec ces canaux n'a été identifiée à ce jour.

Il existe donc un réel besoin de nouvelles molécules utilisables pour le traitement des troubles psychiatriques, notamment de la dépression, molécules plus efficaces, mieux tolérées et ayant une action plus rapide.

### Exposé de l'invention

La présente invention a précisément pour rôle de répondre au besoin et de résoudre les inconvénients de l'art antérieur.

La maturation du NTSR3 libère un peptide, appelé propeptide, qui devient un ligand de ce même type de récepteur. De façon intéressante, ce propeptide est également capable de bloquer l'activité du canal TREK-1.

En effet, le NTSR3 est synthétisé sous la forme d'un précurseur (proNTR3-sortiline). La maturation de ce précurseur, effectuée par la furine, libère un peptide (propeptide) de 44 acides aminés dont la séquence est la suivante :

Ce peptide est capable de se lier au récepteur (NTSR3) maturé. Des études de relations structure-fonction montrent qu'une partie de 17 acides aminés de ce peptide porte toute l'activité de liaison sur le récepteur. Cette partie du peptide est la suivante :

Cette séquence SEQ ID N°2 correspond à la partie soulignée du grand peptide. C'est ce peptide (que nous avons nommé propeptide) qui a été utilisé dans les expérimentations décrites ci-après.

La présente invention se rapporte donc au peptide de séquence ID N°2 . Les «fragments« et «dérivés« de ce peptide sont ceux que l'homme du métier peut aisément déduire des séquences ID n°1 ou 2 annexées, par exemple en remplaçant des acides aminés par leur(s) équivalent(s) ou en raccourcissant le peptide sans que son activité en soit modifiée.

Ces «fragments » ou «dérivés » sont tels qu'ils conservent la propriété de ligand du récepteur 3 de la neurotensine (NTSR3) que possède le peptide de la présente invention. Dans la description qui suit, le peptide de l'invention sera appelé « peptide » ou « propeptide ». Les «dérivés ou fragments« peuvent également être considérés comme étant des analogues du peptide de la présente invention.

Le temps de demi-vie *in vivo* de ce peptide n'est pas encore établi, cependant afin d'améliorer la stabilité ou la biodisponibilité de ce peptide, les inventeurs ont déjà envisagé des modifications sur chacun des acides aminés ainsi que sur la nature des liaisons entre chaque acide aminé. Les modifications sont par exemple les suivantes :
- remplacement d'au moins un acide aminé par un autre de la même famille (aromatique, hydrophobe, basique, etc..) ;
- remplacement d'un acide aminé naturel (amino acide L) par le même acide aminé sous forme D ;
- remplacement d'une liaison peptidique entre deux acides aminés par une liaison pseudo-peptidique.

Ces modifications génèrent en effet des peptides qui sont résistants à l'attaque protéolytique des peptidases et protéases.

La présente invention se rapporte également à une séquence d'acide nucléique codant un peptide . Numéros d'accession GenBank pour les ARNmessagers de la sortiline SORT 1, chez la souris : NM_019972, chez l'Homme : NM_002959. Cette séquence peptidique est par exemple la séquence référencée SEQ ID N°2 sur la liste des séquences annexée. Il peut s'agir de toute séquence appropriée codant le peptide de la présente invention. Cette séquence est de préférence utilisable pour fabriquer le peptide de la présente invention par transfection.

La présente invention se rapporte également à un vecteur comprenant une séquence d'acide nucléique selon l'invention. Il peut s'agir de tout vecteur approprié pour la transformation d'une cellule hôte en vue de faire fabriquer par ladite cellule, par une technique de recombinaison génétique, le peptide de la présente invention. Le vecteur peut être obtenu à partir d'un vecteur choisi, par exemple, dans le groupe comprenant pIRES et pIRES2 et leurs dérivés, pcDNA3 et ses dérivés, pGEX et ses dérivés.

La présente invention se rapporte donc également à une cellule hôte comprenant un peptide présente invention et/ou une séquence d'acide nucléique selon la présente invention et/ou un vecteur selon la présente invention. La cellule hôte peut être toute cellule appropriée pour être transformée et fabriquer ledit peptide de l'invention. Il peut s'agir par exemple de cellules COS-7, HEK 293 et dérivées, N1E115 et apparentées.

La présente invention se rapporte donc également à un procédé de production d'un peptide, comprenant les étapes suivantes :
- transfecter une cellule hôte avec un acide nucléique de l'invention ou transformer une cellule hôte avec un vecteur de l'invention ;
- cultiver ladite cellule hôte dans des conditions permettant l'expression du peptide de séquence ID N°2 ; et
- récupérer ledit peptide de séquence ID N°2 .

Les techniques de transfection et de transformation utilisables pour fabriquer le peptide de la présente invention sont celles connues de l'homme du métier, par exemple celles décrites dans Krieger DE, Erickson BW, Merrifield RB. «Affinity purification of synthetic peptides » Proc Natl Acad Sci U S A.; 73: 3160-3164 (1976) **(16)**.

Selon l'invention, le procédé de fabrication préféré du peptide est la synthèse chimique, par exemple en phase solide. Toute technique connue de l'homme du métier peut être utilisée. Par exemple, le peptide peut être synthétisé selon la technique en phase solide de Krieger DE, Erickson BW, Merrifield RB. «Affinity purification of synthetic peptides » Proc Natl Acad Sci U S A.; 73: 3160-3164 (1976) **(16)**.

La molécule de la présente invention ouvre une nouvelle voie dans la mise en place d'une nouvelle classe d'antidépresseurs et de nouvelles stratégies thérapeutiques des maladies psychiatriques.

La présente invention se rapporte donc également à l'utilisation d'un peptide selon l'invention comme médicament.

En particulier, la présente invention se rapporte à l'utilisation de ce peptide pour la fabrication d'un médicament destiné au traitement de troubles psychiatriques, par exemple pour la fabrication d'un antidépresseur et/ou d'un anti-douleur.

Le peptide de la présente invention tel que défini ci-dessus, est un produit naturel qui constitue un nouveau type d'antidépresseur. Il permet d'éviter tous les effets secondaires non désirables des médicaments actuellement utilisées pour le traitement des troubles psychiatriques. Les effets secondaires des médicaments utilisés dans l'art antérieur proviennent de leur nature chimique. Ces médicaments de l'art antérieur sont des molécules qui ont souvent la propriété de passer les membranes plasmiques cellulaires de manière passive, ce qui entraîne des interactions non spécifiques avec des effecteurs intracellulaires. Le caractère peptidique ou pseudo-peptidique du peptide de la présente invention permet d'éviter ce problème.

Par ailleurs, l'action directe du peptide de la présente invention sur le canal potassique TREK-1 pourrait bien diminuer les délais d'action, souvent longs d'un antidépresseur classique (voir art antérieur ci-dessus).

En outre, le canal TREK-1 est sensible à l'étirement, à l'osmol'arité et à la température. Les inventeurs ont démontré que TREK-1 est l'un des censeurs moléculaires impliqués dans la perception de la douleur. Il est très exprimé dans les neurones sensoriels de petites tailles des ganglions de la racine dorsale de la moelle épinière, il est présent à la fois dans les neurones peptidergiques et non peptidergiques et est colocalisé avec TRPV1, un canal ionique non sélectif activé par la capsaïne et impliqué dans l'hyperalgésie thermique. Les souris KO (« knock-out ») sont plus sensibles aux sensations de douleur et à la chaleur. Ce phénotype est localisé sur les fibres-C polymodales, qui sont plus sensibles à la chaleur. Les souris KO sont aussi plus sensibles à des stimuli mécaniques de bas seuil et montrent une hyperalgésie thermique et mécanique accrue en conditions d'inflammation. Ce travail, publié dans Alloui A, Zimmermann K, Mamet J, Duprat F, Noël J, Chemin J, Guy N, Blondeau N, Voilley N, Rubat-Coudert C, Borsotto M, Romey G, Heurteaux C, Reeh P, Eschalier A, Lazdunski M. «TREK-1, a K+ channel involved in polymodal pain perception » EMBO J; 25 : 2368-2376 (2006) **(17)** désigne le canal TREK-1 comme une cible très intéressante pour le développement de nouveaux analgésiques. Par conséquent toute molécule active sur ces canaux TREK-1 peut avoir des retombées thérapeutiques importantes dans le domaine de la nociception.

En outre le NTSR3 est impliqué dans l'inflammation. Les inventeurs ont démontré que le NTSR3 était responsable des effets de la neurotensine (NT) sur la migration et la libération de cytokines inflammatoires des cellules microgliales (voir Martin S, Vincent JP, Mazella J. «Involvement of the neurotensin receptor-3 in the neurotensin-induced migration of human microglia » J Neurosci. 23 : 1198-1205 (2003) **(18)** et Martin S, Dicou E, Vincent JP, Mazella. J. «Neurotensin and the neurotensin receptor-3 in microglial cells » J Neurosci Res.; 81: 322-326 (2005) **(19)**.

Le peptide de la présente invention possède la propriété d'antagoniser les effets de la NT. Par conséquent, toute molécule capable de bloquer les effets pro-inflammatoires de la NT peut avoir des retombées thérapeutiques importantes dans le domaine de l'inflammation cérébrale.

D'autres avantages pourront apparaître à l'homme du métier à la lecture des exemples qui suivent, donnés bien entendu à titre illustratif et non-limitatif en référence aux figures annexées.

### Brève description des figures

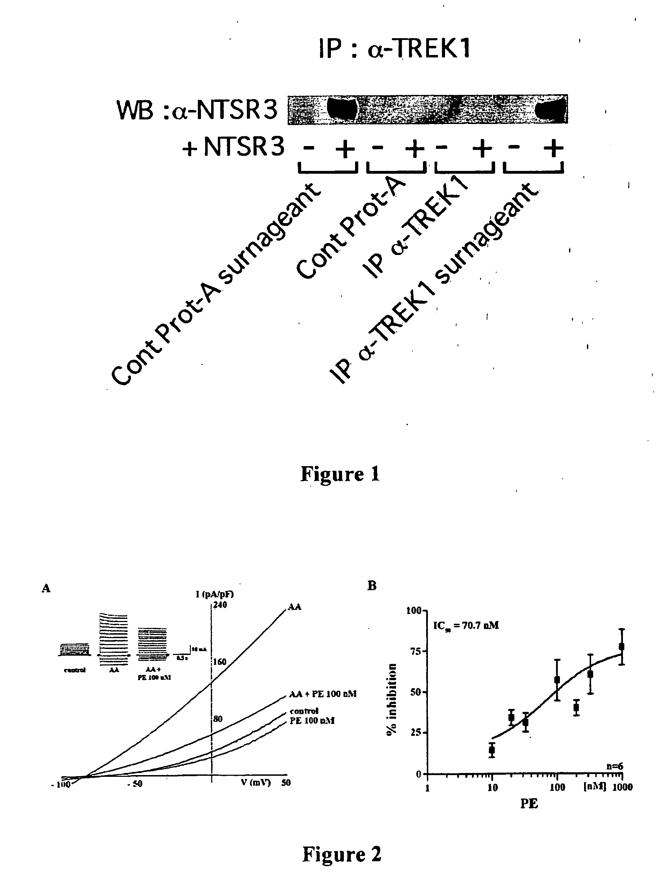

La figure 1 représente un Western blot préparé à partir d'extraits membranaires de cellules COS-7 transfectées avec le canal TREK-1 avec ou sans le récepteur NTSR3, immunoprécipités par un anticorps anti-TREK-1 et où les récepteurs NTSR3/sortiline sont révélés à l'aide d'un anticorps anti-sortiline.

La figure 2 représente des courbes rapportant des résultats expérimentaux de mesures de l'inhibition de l'activité de canaux TREK-1. Sur le graphique A, en abscisse, est représentée la densité du courant 1 (pA/pF) et en ordonnée, la tension appliquée (V), exprimée en mV. En insert, les traces représentant l'amplitude du courant aux différentes valeurs des potentiels. Control : conditions de référence, AA, activation du courant par 10µM d'acide arachidonique, AA + PE, activation du courant par 10µM d'acide arachidonique en présence de 100nM de PE. Sur le graphique B, en abscisse, est représenté le pourcentage d'inhibition du courant TREK-1 activé par 10µM d'AA (% inhibition mesuré à 0 mV) et en ordonnée, la concentration du PE, exprimée en nM. Sur ces figures, pA = picoAmpère et pF = picoFarad.

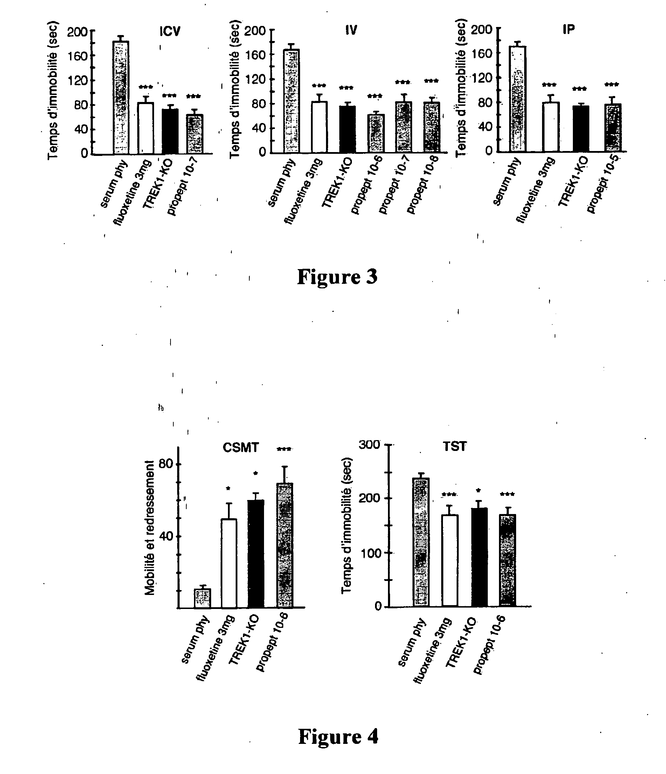

La figure 3 représente des histogrammes rapportant des résultats expérimentaux du test de la nage forcée effectués avec trois modes d'injection différents et avec différentes substances injectées. Il s'agit ici d'un test de résignation. Sur les trois graphiques, en abscisse, le temps pendant lequel l'animal reste immobile (en secondes - sec) et en ordonnée, les différentes substances testées.

La figure 4 représente des histogrammes rapportant des résultats expérimentaux de tests également de résignation CSMT (Conditioned Suppression of Motility : «conditionnement de suppression de mobilité ») et TST (Tail Suspension Test :«test de suspension par la queue ») avec différentes substances injectées. Sur le graphique CMST, en abscisse, le nombre de passages d'une case à l'autre et le nombre de redressements et en ordonnée, les différentes substances testées. Sur le graphique TST, en abscisse le temps pendant lequel l'animal reste immobile (en secondes - sec) et en ordonnée, les différentes substances testées.

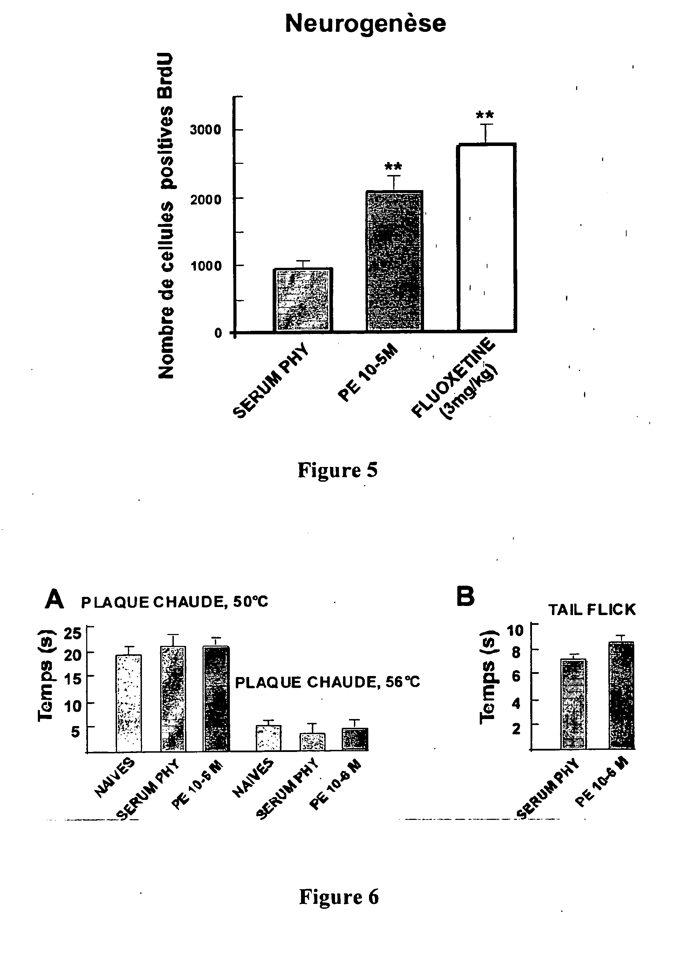

La figure 5 représente un graphique de mesure de la neurogenèse en présence de sérum physiologique, de fluoxétine ou du peptide de la présente invention injecté pendant 2 semaines. En abscisse, le nombre de cellules positives au marquage BrdU. En ordonnée, les substances testées.

La figure 6 représente des histogrammes rapportant des tests de sensibilité à la douleur (à gauche: test de la plaque chaude (Hot plate - à droite test du «tail flick »). Sur les histogrammes A et B, en ordonnée, le temps que la souris met à réagir en secondes et en abscisse, les solutions injectées chez les souris.

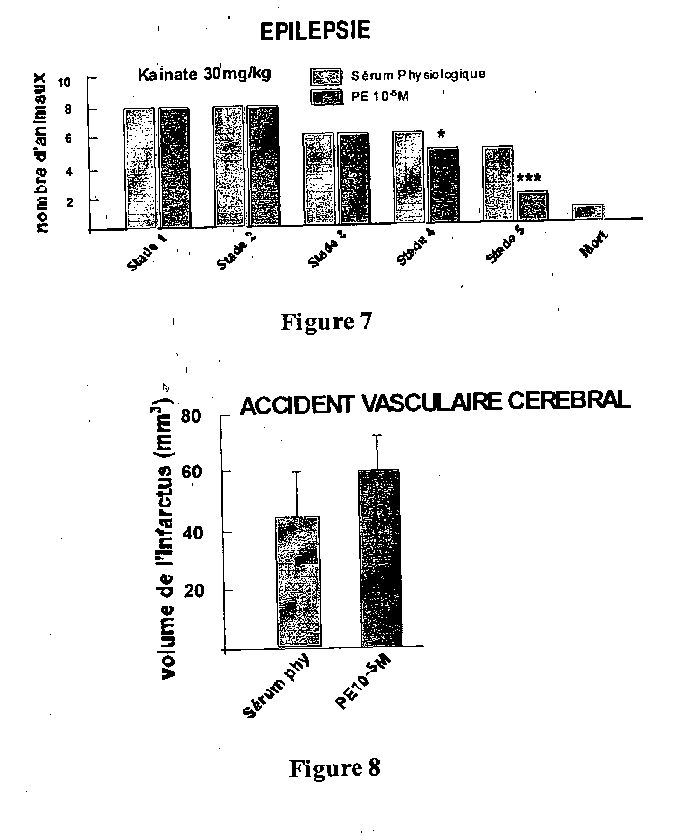

La figure 7 représente un histogramme rapportant les crises épileptiques chez des souris. Sur l'histogramme, l'ordonnée représente le nombre de souris et en abscisse les stades de l'épilepsie. La mesure est faite en fonction de l'injection de kaïnate à 30mg/kg sur des souris traitées avec 100 µl de sérum physiologique ou avec 100 µl d'une solution 10⁻⁵M de peptide («PE 10⁻⁵ M »)

La figure 8 représente un histogramme de quantification de la taille de l'infarctus provoqué par une ischémie focale (modèle d'accident vasculaire cérébral) sur des souris. Sur l'histogramme, l'ordonné représente le volume de l'infarctus et l'abscisse la solution injectée : soit une solution de sérum physiologique («SERUM PHY ») soit une solution de propeptide («PE 10⁻⁵M »).

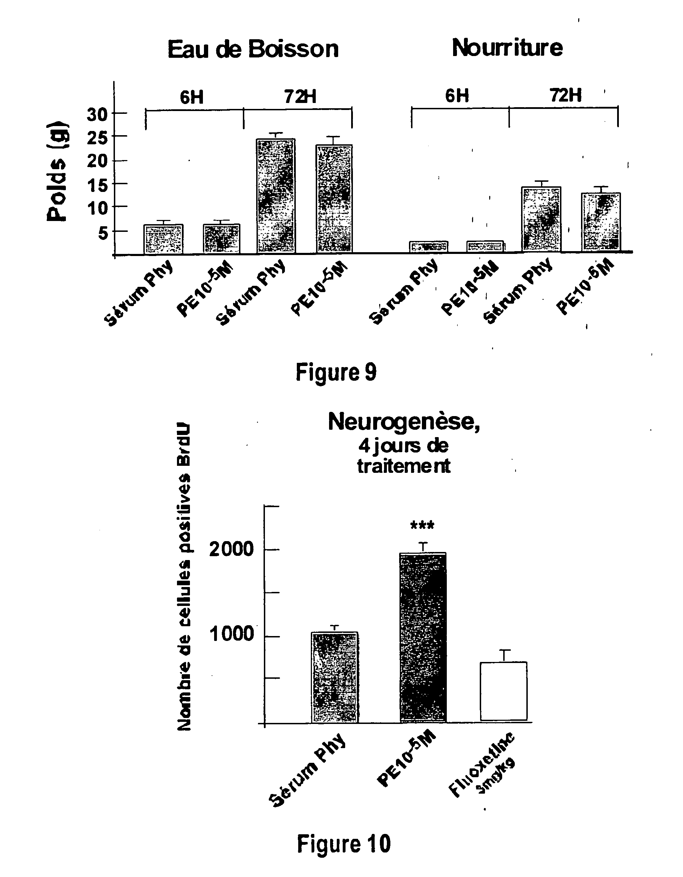

La figure 9 montre un histogramme représentant les prises de boisson et de nourriture à 6 h et 72 h après le dernier traitement chez des souris ayant subi une injection journalière pendant 15 jours. Sur l'histogramme, l'ordonnée représente le poids en gramme et en abscisse les solutions injectées : solution de sérum physiologique («SERUM PHY ») ou une solution de propeptide à une concentration de 10⁻⁵ M («PE 10⁻⁵M »).

La figure 10 montre un histogramme représentant la neurogenèse chez des souris après 4 jours de traitement. Sur l'histogramme, l'ordonné représente le nombre, de cellules positives au Bromodeoxyuridine (BrdU) et en abscisse les solutions injectées : solution de sérum physiologique («SERUM PHY»), une solution de propeptide à une concentration de 10⁻⁵M («PE 10⁻⁵M ») ou une solution comprenant de la fluoxétine à une concentration de 3 mg/kg («FLUOXETINE (3 mg/kg) »).

### EXEMPLES

Les inventeurs ont entrepris des expériences d'électrophysiologie pour démontrer l'inhibition de l'activité des canaux TREK-1 par le propeptide de la présente invention et différents tests dits de comportement visant à valider la propriété antidépresseur du propeptide. En effet, l'équipe du Dr C. Heurteaux a démontré que l'invalidation chez la souris du gène du canal TREK-1 (TREK-1^{-/-} ou KO-TREK-1) conférait aux animaux un phénotype de résistance à la dépression mesuré au travers de tests de comportement reconnus comme étant liés à la dépression **(15)**. Par conséquent, toute molécule capable soit d'inhiber les canaux TREK-1 soit de reproduire un comportement de type TREK-1^{-/-}, peut être considérée comme étant potentiellement antidépressive.

Les inventeurs ont également réalisé des expériences de colocalisation entre TREK-1 et la sortiline sur des coupes de tronc cérébral de souris.

### Exemple 1 : Essais sur TREK-1

L'équipe de Catherine Heurteaux a mis en évidence que le canal potassique TREK-1 pouvait être une cible pour le traitement de la dépression et que des antagonistes de ce canal pouvaient avoir la propriété d'être des antidépresseurs puissants.

TREK-1 est régulé par les neurotransmetteurs qui modulent le niveau d'AMPc via des récepteurs qui activent la voie des protéines Gq comme, le récepteur 5-HT (sérotonine). La délétion de TREK-1 résulte en un phénotype anti-dépression avec une augmentation effective de la neurotransmission 5-HT. Les souris KO-TREK-1 développent un comportement similaire à celui de souris naïves traitées avec les antidépresseurs classiques **(15)**. Ces données suggèrent que des bloqueurs du canal TREK-1, qui n'existent pas aujourd'hui, peuvent mener à une nouvelle génération d'antidépresseurs. Tout ce qui peut affecter le trafic, l'adressage et la fonction du canal TREK-1 est donc important à identifier. Il s'agit de découvrir des partenaires qui modulent ces propriétés.

Les inventeurs de la présente invention démontrent ici qu'un bon candidat est le récepteur-3 de la neurotensine, le NTSR3 également nommé sortiline. Cette protéine multifonctionnelle (NTSR3/sortiline) peut en effet jouer le rôle de récepteur ou de co-récepteur et elle possède plusieurs ligands tels que la neurotensine, la lipoprotéine lipase et le propeptide libéré lors de la maturation du précurseur du NTSR3/sortiline. Ce propeptide est un antagoniste spécifique des effets de la neurotensine. Le NTSR3/sortiline joue également un rôle dans l'adressage d'autres protéines à la surface cellulaire.

Dans le but de voir si le NTSR3/sortiline joue un rôle dans le trafic du canal TREK-1, les inventeurs ont entrepris des expériences visant à rechercher l'existence d'interactions entre les deux protéines. Pour cela, ils ont réalisé des transfections dans les cellules COS-7 avec les deux protéines NTSR3/sortiline et TREK-1.

Les résultats montrent que l'immuno-précipitation des extraits cellulaires avec un anticorps anti-TREK-1 co-précipite le NTSR3/sortiline (Figure 1 annexée). En outre les inventeurs ont démontré une interaction fonctionnelle entre TREK-1 et le NTSR3/sortiline au moyen d'expériences d'électrophysiologie : l'activation de TREK-1 par l'acide arachidonique est bloquée par le propeptide, un inhibiteur spécifique et sélectif du NTSR3/sortiline (Figure 2).

Pour obtenir le gel représenté sur la figure 1, les cellules COS-7 transfectées avec TREK-1 et NTSR3/sortiline ont été lysées. Le surnageant a été incubé avec un anticorps anti-TREK-1 (IP : α-TREK-1) en présence de protéine-A sépharose pendant 16 heures à 4°C.

Les protéines ainsi précipitées sont déposées sur un gel SDS-PAGE puis transférées sur nitrocellulose. La protéine NTSR3/sortiline est détectée à l'aide d'un anticorps anti-NTSR3/sortiline.

### Exemple 2 : Action du propeptide sur l'activité du canal TREK-1

Toutes les expériences de mesure de l'effet du propeptide ont été réalisées sur des cellules COS-7 (lignée cellulaire issue de fibroblastes de rein de singe vert africain Cercopithecus aethiops). Ces cellules sont ensemencées à une densité de 20 000 cellules/boîte de 35mm de diamètre, 24 heures avant d'être transfectées par la méthode du DEAE-dextran avec 1µg de plasmide pIRES-EGFP-TREK-1. Les mesures de courant sont réalisées 48 à 72 heures après la transfection.

Culturel des cellules COS-7. Les cellules COS-7 (référence ATCC : CRL-1651) sont cultivées dans un milieu DMEM (Gibco) / 10% sérum de veau fétal (SVF, ICN), à 37°C en présence de 5% de CO2.

Transfection au DEAE dextran. Au jour 1, les cellules COS-7 sont ensemencées à 20 000 cellules par boîte de diamètre 35mm contenant 2 ml de milieu de culture. Au jour 2, le milieu est éliminé et les cellules sont recouvertes par 200 µl de PBS (Gibco) contenant 1µg de plasmide pIRES-EGFP-TREK-1 et 100 µg de DEAE-dextran (Sigma) puis placées à 37°C/5% CO2. Après 30 minutes d'incubation, on ajoute 2 ml d'un milieu DMEM/ 10% NuSérum/ 80µM chloroquine. Après 3 heures, le milieu est remplacé par 2 ml de DMEM/10% SVF et les cellules sont incubées 48 à 72 heures avant d'être mesurées par des méthodes électrophysiologiques

Mesures électrophysiologiques : toutes les mesures sont faites à température ambiante, c'est-à-dire à 21 - 22°C. Les cellules ayant été transfectées par le plasmide sont repérées grâce à la fluorescence émise par la EGFP après excitation à 480nm.

La technique du patch clamp sur cellule entière a été utilisée pour mesurer l'activité des canaux TREK-1. L'appareillage utilisé est le RK 400 patch-clamp amplifier (Axon Instruments, U.S.A.). Les pipettes de patch de résistance 1,3 à 8 MΩ sont préparées à partir de capillaires en verre borosilicate et sont remplies avec une solution 155 mM KCI, 3 mM MgCl₂,, 5 mM EGTA, 10 mM HEPES/KOH pH 7,2.

Le mileu de culture cellulaire est remplacé par une solution 150 mM NaCl, 5 mM KCI, 3 mM MgCl₂, 1mM CaCl₂, 10 HEPES/ NaOH pH 7,4 contenant 10 mM Chlorure de TétraEthyl Ammonium, 3 mM de 4-Aminopyridine. Les cellules sont perfusées en continu avec cette solution. Le potentiel de repos de la membrane de la cellule mesurée est fixé à -80mV.

Les variations de voltage sont obtenues soit par rampe continue (de - 100 à + 50mV) soit par saut de potentiel de 10mV (de -100 à + 40mV, 1,5 seconde par saut).

Les données obtenues ont été analysées avec le logiciel Pclamp. Les courants décrits dans la figure 2B annexée ont été obtenus à 0 mV, les résultats exprimés sont les moyennes ± la déviation standard. La valeur de l'IC₅₀ a été obtenue en traçant les données expérimentales à l'aide d'une fonction sigmoïdale.

L'activité du canal est ensuite mesurée par la technique de *« patch-clamp »* en configuration cellule entière comme décrit ci-dessus.

Dans des conditions basales, c'est-à-dire en absence d'activation par l'acide arachidonique (connu pour être un activateur puissant du canal TREK-1), 100 µM de propeptide inhibent 25% de l'activité canal mesurée à 0 mV (31 pA/pF contre 23 pA/pF).

Dans les conditions où le canal est activé par 10 µM d'acide arachidonique (31 pA/pF contre 130 pA/pF), 100 µM de propeptide inhibent 67% de l'activité du canal mesurée à 0 mV (130 pA/pF contre 56 pA/pF).

Dans les mêmes conditions expérimentales, une courbe dose-réponse de l'inhibition de l'activité des canaux TREK-1 mesurée à 0 mV a été réalisée et est représentée sur la figure 2B annexée. Elle indique une concentration de demi-effet (IC₅₀) de 70.7 nM.

### Exemple 3 : Tests de comportement

Les inventeurs ont réalisé trois types d'expériences de comportement considérés comme classiques pour déterminer l'activité antidépresseur d'une substance (Nestler E.J., Gould E., Manji H., Buncan M., Duman R. S., Greshenfeld H. K., Hen, R. et al. «Preclinical models: status of basic research in depression« Biol Psychiatry. 15, 503-528 (2002) **(20)** et Cryan, J. & Holmes, A. «The ascent of mouse: advances in modeling human depression and anxiety » Nat Rev Drug Discov. 4, 775-790 (2005) **(21)).** Les effets du propeptide de la présente invention ont été comparés à ceux de la solution saline dans laquelle il est dissous et à ceux de la fluoxétine, un antidépresseur des plus utilisés en clinique. La souche de souris utilisée est la souche C57Black/J. Des souris TREK-1^{-/-} ont également été mesurées dans ces différents tests.

Le propeptide est dissous dans une solution saline 0,9% NaCl aux concentrations désirées : 10⁻⁵ M, 10⁻⁶ M, 10⁻⁷ M et 10⁻⁸ M. Différents volumes sont injectés en fonction de la technique utilisée :
- en intra cérébro ventriculaire : 5 µl
- en intraveineuse : 100 µl
- en intrapéritonéale : 100 µl

Pour les injections ICV une seringue Hamilton (Fisher Bioblock)(marque de commerce) de 10 µl est utilisée, et dans le cas des injections IV ou IP c'est une aiguille de 0,45 x 12 mm qui est utilisée.

### A. Test de la nage forcée (ou Forced Swimming Test, FST) (Cryan & Holmes, 2005 (21))

L'expérience consiste, 30 min après injection de la substance à tester, à plonger la souris pendant 6 minutes dans un récipient de 15 cm de diamètre, de 30 cm de hauteur contenant 11 cm d'une eau à 22°C et de mesurer le temps d'immobilité lors des 4 dernières minutes. Les souris traitées avec des antidépresseurs ont des temps d'immobilité inférieurs à ceux des souris contrôle. Les inventeurs ont testé différentes voies d'injection du peptide et de la solution saline : intra-cérébro-ventriculaire (ICV), intraveineuse (IV) ou intrapéritonéale (IP). Seules des injections IP ont été utilisées pour administrer la fluoxétine. Un contrôle avec une injection de sérum physiologique a été réalisé.

Les résultats de ces tests sont représentés sur la figure 3 annexée : Tests FST, les valeurs ± SEM sont statistiquement comparées à la condition contrôle (sérum physiologique) *** p< 0,001, (Test «NOVA« à 2 facteurs = test d'analyse de variances suivi d'un test post-hoc).

Quelle que soit la voie d'administration, les effets du propeptide de la présente invention sont comparables à ceux provoqués par la fluoxétine. Les souris traitées par le propeptide ont un comportement, identique aux souris KO-TREK-1.

Dans les deux tests suivants, le propeptide de la présente invention a été administré par voie IV à raison de 100µl à 1µM par animal.
B. Test de la suspension par la queue (ou « Tail Suspension Test », TST) (voir Cryan, J. & Holmes, A. (2005) **(21)** et Ripoll, N., David, D., Dailly, E., Hascoet, M. & Bourin, M. «Antidepressant-like effects in various mice strains in the tail suspension« Behav Brain Res. 143(2:193-200 (2003) **(22).)**

L'expérience consiste, 30 min après injection de la substance testée à la souris, à suspendre la souris par la queue à l'aide d'un ruban adhésif et à mesurer le temps d'immobilité pendant 6 min.

Comme indiqué sur le graphique de la figure **4** annexée (TST), les souris traitées par des antidépresseurs ont des temps d'immobilité diminués par rapport aux souris contrôles.

Dans cette expérimentation encore, les effets du propeptide de la présente invention sont comparables à ceux provoqués par la fluoxétine, et les souris traitées par le propeptide ont un comportement identique aux souris TREK-1^{-/-} (figure **4****,** TST)).

Le propeptide est dissous dans une solution saline 0,9% NaCl aux concentrations désirées : 10⁻⁵ M, 10⁻⁶ M, 10⁻⁷ M et 10⁻⁸ M. Différents volumes sont injectés en fonction de la technique utilisée :
- en intra cérébro ventriculaire : 5 µl
- en intraveineuse: 100 µl
- en intrapéritonéale : 100 µl

Pour les injections ICV une seringue Hamilton (Fisher Bioblock)(marque de commerce) de 10 µl est utilisée, et dans le cas des injections IV ou IP c'est une aiguille de 0,45 x 12 mm qui est utilisée.
**C. Test de suppression de mobilité conditionnée (ou « Conditioned Motility Suppression Test », CMST)** (voir Daugé, V., Sebret, A., Beslot, F., Matsui, T., & Roques B. «Behavorial profile of CCK2 receptor-deficient mice »Neuropsychophamacol; 25: 690-698 (2001) (23).

L'expérience consiste à conditionner, au jour 1, la souris et, au jour 2, mesurer les effets des substances.

Jour 1, chaque souris est placée dans une boîte où le plancher est une grille métallique reliée à un stimulateur électrique. Le conditionnement consiste à générer 30 séquences constituées d'un choc électrique (200ms à 1,8 mA) suivi de 12 secondes de latence (temps total de conditionnement : 6 minutes). Les souris sont ensuite remises en cage et laisser à récupérer sur 24 heures.

Jour 2, les souris sont injectées avec les différentes substances testées puis après 30 minutes, elles sont replacées dans la boîte qui a servi au conditionnement et où le plancher a été divisé en 6 cases identiques. Aucun choc électrique n'est délivré.

La mesure s'effectue en comptant manuellement pendant 6 minutes le nombre de fois où chaque souris change de case ou se redresse.

Les souris choquées non traitées (sérum physiologique) ne bougent pratiquement pas («*freezing* »), les souris choquées traitées récupèrent une mobilité importante par rapport à ces dernières : 5 fois supérieure avec la fluoxétine (à raison d'une injection de 3 mg/kg) et 7 fois supérieure avec le propeptide de la présente invention.

Les résultats expérimentaux de ce test sont représentés sur la figure 4 annexée. Les souris TREK-1^{-/-} ont une mobilité 6 fois supérieure au contrôle (CSMT sur la figure 4).

L'ensemble de ces résultats expérimentaux démontrent clairement que le propeptide de la présente invention possède des propriétés d'antidépresseur au moins aussi efficaces que celles de la fluoxétine voire supérieures dans le cas du test «conditionnement de suppression de mobilité ».

De plus, le fait que le propeptide de la présente invention agisse par voies intraveineuse ou intrapéritonéale aussi bien que par voie intracisternale indique qu'il passe facilement la barrière hémato-méningée (BHE) pour exercer ses effets. Cette propriété ouvre la possibilité de traitements chroniques et facilement transposables pour des tests cliniques.

### Exemple 4 : Effets du propeptide sur la neurogenèse

Les traitements par les antidépresseurs sont connus pour augmenter la neurogenèse dans l'hippocampe (Santarelli, L., Saxe, M, Gross, C, Surget, A, Battaglia, F, Dulawa, S, Weisstaub, N, Lee, J, Duman, R, Arancio, O, Belzung, C, Hen, R et al «Requirement of hippocampal neurogenesis for the behavioral effects of antidepressants » Science, 301 : 805-809 (2003) **(24).** Cette prolifération cellulaire est mesurée par l'augmentation de cellules «progénétrices » qui incorporent un marqueur le 5-bromo-2-deoxyuridine (BrdU) et se transforment en neurones matures. La détection du BrdU se fait par immunohistochimie.

Au jour 1, les animaux sont injectés en IP avec 300 µl d'une solution aqueuse de 10mM de BrdU, puis remis en cage.

Au jour 2, les animaux sont euthanasiés puis les tissus sont fixés grâce à la perfusion intracardiaque d'une solution froide de 4% paraformaldéhyde. Les cerveaux sont prélevés puis congelés à -20°C.

Des coupes sériées de 40µm sont préparées, de façon à recouvrir la totalité de la structure hippocampe, à l'aide d'un vibratome (Leica). Une coupe sur six est retenue pour mesurer l'incorporation de BrdU à l'aide d'un anticorps anti-BrdU monoclonal de souris (Becton Dickinson). Cet anticorps primaire est révélé grâce à un anticorps secondaire couplé à la biotine et à de l'avidine-peroxidase. L'activité peroxidase est visualisée par hydrolyse du DAB. Les cellules ainsi marquées sont comptées sur chaque coupe.

Pour chaque groupe, solution saline, propeptide ou fluoxétine, les nombres de cellules marquées au BrdU de chaque coupe sont additionnés et multipliés par, six de façon à considérer l'ensemble de la structure. Chaque groupe est constitué de 3 souris, les résultats exprimés sont la moyenne des trois animaux.

Les résultats obtenus sont représentés sur l'histogramme de la figure 5 annexée. Ce graphique indique la mesure de la neurogenèse. Le sérum physiologique («SERUM PHY ») et le propeptide 10µM («PE 10⁻⁵M ») ont été injectés pendant 2 semaines, la fluoxétine a également été administrée par l'eau de boisson, solution à 80 mg/l pendant 2 semaines, période démontrée nécessaire pour obtenir une efficacité de la fluoxétiné . ** p< 0,01, * p< 0,05.

Ces résultats démontrent clairement que le propeptide induit une neurogenèse importante comparable à celle induite par la fluoxétine. In n'y aucune différence significative entre les mesures obtenues avec le propeptide et celles obtenues avec la fluoxétine (Test ANOVA à un facteur suivi du test post-hoc)_{.}

### Exemple 5 : Effets du propeptide sur la douleur

Le peptide de l'invention inhibe l'activité des canaux TREK-1. Les principaux effets secondaires envisageables sont ceux induits par le blocage de l'activité des canaux TREK-1. En effet, des travaux de l'équipe de C. Heurteaux (Heurteaux C., Guy, N.,Laigle, C.,Blondeau, N.,Duprat, F.,Mazzuca, M.,Lang-Lazdunski, L., Widmann, C. et al «TREK-1, a K(+) channel involved in neuroprotection and general anesthesia » EMBO J, 23 : 2684-2695 (2004) **(25)** et Alloui A, Zimmermann K, Mamet J, Duprat F, Noël J, Chemin J, Guy N, Blondeau N, Voilley N, Rubat-Coudert C, Borsotto M, Romey G, Heurteaux C, Reeh P, Eschalier A, Lazdunski M. «TREK-1, a K+ channel involved in polymodal pain perception » EMBO J; 25 : 2368-2376 (2006)) (17) ont montré que les souris invalidées pour le canal TREK-1 (TREK-1 ^{-/-}) étaient :
- plus sensibles à la douleur que les souris sauvages (Alloui A, Zimmermann K, Mamet J, Duprat F, Noël J, Chemin J, Guy N, Blondeau N, Voilley N, Rubat-Coudert C, Borsotto M, Romey G, Heurteaux C, Reeh P, Eschalier A, Lazdunski M. «TREK-1, a K+ channel involved in polymodal pain perception » EMBO J; 25: 2368-2376 (2006)) **(17).**
- les effets neuroprotecteurs des acides gras polyinsaturés, comme l'acide alpha linolénique, dans le cas d'une ischémie cérébrale disparaissaient chez les souris KO, et
- que les crises d'épilepsie induites par une injection de kaïnate étaient beaucoup plus sévères et conduisaient beaucoup plus souvent à la mort chez les souris TREK-1 ^{-/-} (Heurteaux C., Guy, N., Laigle, C., Blondeau, N., Duprat, F., Mazzuca, M., Lang-Lazdunski, L., Widmann, C. et al «TREK-1, a K(+) channel involved in neuroprotection and general anesthesia » EMBO J, 23 : 2684-2695 (2004)) **(25).**

La sensibilité à la douleur a été mesurée avec deux tests: le test de la plaque chaude et le test de «tail flick ».

### 1. La plaque chaude (hot plate).

Ce test consiste à mesurer le temps que met une souris pour se lécher les pattes arrières lorsqu'elle est posée sur une plaque maintenue à une température donnée. Deux températures ont été utilisées : 50 et 56°C.

Les souris utilisées étaient des souris C57BL6/J. Chaque groupe était constitué de 10 souris.

Le premier groupe de souris «naïves » n'a reçu aucune injection, le second groupe de souris a reçu une injection intraveineuse de 100 µl de sérum physiologique («SERUM PHY »), enfin le troisième groupe a reçu une injection de 100 µl de solution comprenant le peptide de l'invention à une concentration de 10⁻⁶ M («PE 10⁻⁶M »).

Une mesure de la sensibilité à la chaleur a ensuite été effectuée sur les trois groupes de souris et une comparaison des résultats obtenus a été effectuée.

Les mesures ont été effectuées 30 minutes après injection.

Les résultats sont présentés dans la Figure 6A.

Ces résultats démontrent clairement qu'il n'y aucune différence statistique entre les trois groupes d'animaux à 50°C: naïves : 19,4 +/- 1,6 sec, injectées sérum physiologique : 21,3 +/- 2,1 sec et injectées PE : 21,1+/- 1,5 sec. A 56°C, naïves : 5,3 +/- 1,6 sec, injectées sérum physiologique : 3,8 +/-0,7 sec et injectées PE : 4,6 +/- 0,9 sec.

La mesure de la différence statistique a été effectuée en utilisant le test ANOVA un facteur suivi du test post-hoc.

Cet exemple démontre clairement que le peptide de l'invention n'induit pas d'hyperalgésie.

### 2. 2. Le « tail flick »

Ce test consiste à faire tremper la queue d'une,souris dans un bain à 48°C et à mesurer le temps qu'elle met à la retirer du bain. Avant toute injection, un test d'habituation a été effectué sur les souris. Il consiste à plonger la queue de chaque animal deux fois dans le bain de façon à avoir des groupes homogènes avant tout traitement (obtention d'un temps de réaction inférieur à 12 secondes). Puis 100 µl de sérum physiologique («SERUM PHY ») ou 100 µl de peptide de séquence ID N°2 à une concentration de 10⁻⁶ M («PE 10⁻⁶M ») ont été injectés chez des souris.

Les souris utilisées étaient des souris C57BL6/J. Chaque groupe était constitué de 10 souries.

Les mesures ont été effectuées 30 minutes après injection. Les résultats présentés dans la Figure 6B indiquent qu'il n'y a pas de différence significative entre les deux groupes de souris : sérum physiologique : 7,0+/- 0,6 sec et PE : 8,3 +/- 0,7 sec.

L'évaluation de la différence a été effectuée en utilisant le Student t test. Ce test a confirmé qu'il n'y avait pas de différence significative entre les deux groupes.

Le test de la plaque chaude et du «tail flick » démontrent clairement que l'injection du peptide de séquence ID N°2 n'induit pas d'hyperalgésie.

### Exemple 6 : Effets du propeptide sur l'épilepsie

Dans cette expérience, les souris utilisées étaient des C57BL6/J à raison de 10 souris par groupe expérimental: un groupe «Sérum Physiologique », qui a reçu une injection intrapéritonéale de 100 µl de sérum physiologique et un groupe « PE 10⁻⁵ M », dans lequel les souris ont reçu une injection intrapéritonéale de 100 µl d'une solution comprenant le peptide de l'invention à une concentration de 10⁻⁵M. Dans chacun des groupes expérimentaux, les crises épileptiques ont été induites par injection intrapéritonéale de 100 µl de kaïnate à raison de 30mg/kg. L'observation du stade de la crise d'épilepsie a été effectuée 90 minutes après l'injection de Kaïnate. Cette observation a été faite par observation du comportement des souris.

Les différents stades de la crise épileptique sont exposés dans le document Tsirka S. E., Gualandris, A., Amaral, D. G., Strickland, S. «Excitotoxin-induced neuronal degeneration and seizure are mediated by tissue plasminogen activator », Nature, 377 : 340-344 (1995) **(26).**

6 stades sont définis pour décrire l'intensité de la crise épileptique :
Stade 1 : immobilité
Stade 2 : mouvements de la tête et de la nuque
Stade 3 : activité clonique unilatérale
Stade 4 : activité clonique bilatérale
Stade 5 : convulsions généralisées
Stade 6 : mort de l'animal

Les résultats présentés dans la Figure 7 montrent que non seulement le peptide de l'invention n'aggrave pas les crises induites par le kaïnate mais qu'au contraire il peut avoir un effet bénéfique puisque seuls 2 animaux injectés avec du peptide ont atteint le stade 5 contre 5 dans le groupe «Sérum Physiologique ». De plus le seul animal mort était dans le groupe «Sérum Physiologique ».

L'analyse statistique a été faite par un test ANOVA à 2 facteurs suivi du test post-hoc.

Cet exemple démontre donc clairement que le propeptide n'induit pas de crises épileptiques et qu'au contraire il présente un effet protecteur vis- à-vis de l'épilepsie.

### Exemple 7 : Effets du propeptide sur les risques d'Accident Vasculaire Cérébral (AVC)

Le modèle utilisé pour induire un AVC est celui de l'ischémie focale, qui consiste en l'occlusion par un filament de l'artère cérébrale moyenne (Heurteaux C, Laigle, C., Blondeau, N., Jarretou, G., Lazdunski, M. «Alpha-linolenic acid and riluzole treatment confer cerebral protection and improve survival after focal brain ischemia » Neuroscience, 137 : 241-251 (2006) **(27)**. La taille des infarctus provoqués par cette ischémie focale a été mesurée après coloration au crésyl violet des coupes de cerveaux. La mesure de l'infarctus se fait en traçant le contour de la zone infarcie par un système d'analyse d'image (Olympus DP Soft) selon la méthode décrite dans Heurteaux *et al,* 2006 **(27)**. Les cerveaux de souris C57BL6/J (à raison de 10 souris par groupe expérimental) sont traitées pendant une semaine avec une injection intrapéritonéale journalière de 100µl d'une solution comprenant soit le peptide de séquence ID N°2 à une concentration de 10⁻⁵M (groupe «PE 10⁻⁵M »), soit avec d'une solution de sérum physiologique (groupe «SERUM PHY »). Les valeurs ont été comparées entre les deux groupes.

Les résultats présentés dans Figure 8 démontrent clairement qu'il n'y a pas de différence significative entre les deux groupes d'animaux.

L'évaluation de la différence a été effectuée en utilisant le test *t* de Student Ce test a confirmé qu'il n'y avait pas de différence significative entre les deux groupes.

Cet exemple démontre clairement que le peptide n'a pas d'effet significatif sur la taille des infarctus provoqués par une ischémie focale. Cet exemple démontre donc clairement que le peptide de séquence ID N°2 n'induit pas une augmentation des dégâts neuronaux et n'augmente pas le risque d'accident vasculaire cérébral.

### Exemple 8 : Effets du propeptide sur la prise alimentaire

La prise de nourriture et de boisson a également été mesurée. Deux groupes expérimentaux de souris C57BL6/J (n=10 par groupe) ont été utilisés : le premier groupe recevant une injection intrapéritonéale journalière pendant 15 jours de 100µl de sérum physiologique («SERUM PHY »), le second groupe recevant une injection intrapéritonéale journalière pendant 15 jours de 100 µl d'une solution comprenant le peptide de séquence ID N°2 à une concentration de 10⁻⁵M («PE 10⁻⁵ M »).

Ces mesures ont été effectuées 6 et 72 heures après la fin du traitement chronique. Il n'y a aucune différence significative entre les animaux traités et les non traités aussi bien pour la prise de nourriture que pour celle de la boisson (Figure 9).

L'évaluation de la différence a été effectuée en utilisant le test *t* de Student. Ce test a confirmé qu'il n'y avait pas de différence significative sur la prise de nourriture et de boisson entre les deux groupes.

Cet exemple démontre clairement que le peptide de séquence ID N°2 n'a pas d'effet significatif sur la prise alimentaire des animaux.

### Exemple 9 : Mesure du délai d'action du propeptide

A l'inverse de nombreux antidépresseurs, couramment utilisés aujourd'hui, l'efficacité d'action du peptide de l'invention est très raphide. En effet, un des moyens expérimentaux pour démontrer l'efficacité des antidépresseurs est de mesurer la néoneurogenèse (Santarelli, L.; Saxe, M, Gross, C, Surget, A, Battaglia, F, Dulawa, S, Weisstaub, N, Lee, J, Duman, R, Arancio, O, Belzung, C, Hen, R et al «Requirement of hippocampal neurogenesis for the behavioral effects of antidepressants » Science, 301 : 805-809 (2003) **(24)**. En effet, les antidépresseurs tels que la fluoxétine, induisent une neurogenèse au bout d'environ deux semaines. Cette neurogenèse est un élément clé dans le mécanisme d'action des antidépresseurs. En clinique, il est démontré que la dépression est associée à une atrophie des cellules pyramidales de l'hippocampe Sheline, Y. I.,Wang, P. W., Gado, M. H., Csernansky, J. G.,Vannier, M. W. «Hippocampal atrophy in recurrent major depression » Proc Natl Acad Sci U S A, 93 : 3908-3913 (1996) **(28)** et à une forte diminution de la neurogenèse dans le dentate gyrus (Gould, E., Tanapat, P., McEwen, B. S.,Flugge, G., Fuchs, E. «Proliferation of granule cell precursors in the dentate gyrus of adult monkeys is diminished by stress » Proc Natl Acad Sci U S A, 95: 3168-3171 (1998)) **(29).**

Le protocole expérimental est celui décrit dans le matériel et méthodes (Supplement) de la publication Santarelli et al, «Requirement of hippocampal neurogenesis for the behavioral effects of antidepressants » Science, 301 : 805-809 (2003) **(24)** à l'exception de la durée de traitement, qui est de quatre jours.

Les résultats de la Figure 10 annexée indiquent une grande rapidité d'action du peptide dans l'induction de la neurogenèse. Il y a une augmentation significative de la neurogenèse qu'avec le peptide («PE 10⁻⁵ M »). La neurogenèse induite par la fluoxétine («FLUOXETINE, 3 mg/kg ») est identique à celle induite par l'injection de sérum physiologique («SERUM PHY ») durant cette période de 4 jours.

L'évaluation de l'augmentation a été effectuée en utilisant le test ANOVA à un facteur suivi du test post-hoc). Ce test a confirmé que par rapport au groupe «Sérum Phy » il y avait une augmentation significative de la neurogenèse induite par le peptide de l'invention mais pas par la fluoxétine.

Les exemples démontrent donc clairement que le, peptide de l'invention peut être utilisé pour soigner les maladies psychiatriques, les phénomènes inflammatoires et/ou douloureux. De plus, le peptide de l'invention n'induit pas les effets secondaires connus des antidépresseurs de l'état de la technique et a un délai d'action inférieur aux antidépresseurs de l'état de la technique, c'est-à-dire une rapidité supérieure d'action.

### Liste des references

(1) Wong, M. & Licinio, J. Research and treatment approaches to (1) Wong, M. & Licinio, J. Research and treatment approaches to depression. Nat Rev Neurosci., 2, 343-351 (2001).
(2) Moller HJ. Suicide, suicidality and suicide prevention in affective disorders. Acta Psychiatr Scand; 418 (suppl) : 73-80 (2003).
(3) Nestler E. Barrot M. , DiLeone R. J.,Eisch A. J.,Gold S. J.,Monteggia, L. M.Neurobiology of depression. Neuron 34, 13-25 (2002).
(4) Baghai TC, Volz HP, Moller HJ. Drug treatment of depression in the 2000s: An overview of achievements in the last 10 years and future possibilities. World J Biol Psychiatry, 7:198-222 (2006).
(5) Weilburg JB. An overview of SSRI and SNRI therapies for dépression. Manag Care. Jun; 13 (6 Suppl Depression): 25-33 (2004).
(6) Manji HK, Gottesman II, Gould TD. Signal transduction and genes-to-behaviors pathways in psychiatric diseases. Sci STKE; 207 : pe49 (2003).
(7) Reus VI, Wolkowitz OM. Antiglucocorticoid drugs in the treatment of depression. Expert Opin Investig Drugs ; 10 : 1789-1796 (2001).
(8) Griebel G, Simiand J, Steinberg R, et al. 4-(2-Chloro-4-methoxy-5-methylphenyl)-N-[(1S)-2-cyclopropyl-1-(3-fluoro-4-methylphenyl)ethyl]5-methyl-N-(2-propynyl)-1, 3- thiazol-2-amine hydrochloride (SSR125543A), a potent and selective corticotrophin-releasing factor(1) receptor antagonist. II. Characterization in rodent models of stress-related disorders. J Pharmacol Exp Ther ; 301 : 333-345 (2002).
(9) Kramer MS, Cutler N, Feighner J, et al. Distinct mechanism for antidepressant activity by blockade of central substance P receptors. Science ; 281 : 1640-1645 (1998).
(10) Skolnick P. Antidepressants for the new millennium. Eur J Pharmacol ; 375 : 31-40 (1999).
(11) Kempermann G, Kronenberg G. Depressed new neurons. Adult hippocampal neurogenesis and a cellular plasticity hypothesis of major dépression. Bio/ Psychiatry ; 54 : 499-503 (2003).
(12) Malberg JE, Schecter LE. Increasing hippocampal neurogenesis: a novel mechanism for antidepressant drugs. Curr Pharm Des; 11 : 145-155 (2005).
(13) Duman, R. & Monteggia, L. A neurotrophic model for stress-related mood disorders. Biol Psychiatry ; 9, 1116-1127 (2006).
(14) Henn FA, Vollmayr B. Neurogenesis and depression: etiology or epiphenomenon? Biol Psychiatry; 56 : 146-150 (2004).
(15) Heurteaux, Lucas, Guy, El Yacoubi, Thümmler, Peng, Noble, Blondeau, Widmann et al., Deletion of TREK-1, a background potassium channel, results in a depression-resistant phenotype, Nature Neurosci., 9, 1134-1141 (2006).
(16) Krieger DE, Erickson BW, Merrifield RB. Affinity purification of synthetic peptides. Proc Natl Acad Sci U S A.; 73: 3160-3164 (1976).
(17) Alloui A, Zimmermann K, Mamet J, Duprat F, Noël J, Chemin J, Guy N, Blondeau N, Voilley N, Rubat-Coudert C, Borsotto M, Romey G, Heurteaux C, Reeh P, Eschalier A, Lazdunski M. TREK-1, a K+ channel involved in polymodal pain perception. EMBO J. ; 25: 2368-2376 (2006).
(18) Martin S, Vincent JP, Mazella J. Involvement of the neurotensin receptor-3 in the neurotensin-induced migration of human microglia. J Neurosci. ; 3: 1198-1205 (2003).
(19) Martin S, Dicou E, Vincent JP, Mazella J. Neurotensin and the neurotensin receptor-3 in microglial cells. J Neurosci Res.; 81: 322-326 (2005).
(20) Nestler E.J., Gould E., Manji H., Buncan M., Duman R. S., Greshenfeld H. K., Hen, R. et al. Preclinical models: status of basic research in depression. Biol Psychiatry. 15, 503-528 (2002).
(21) Cryan, J. & Holmes, A. The ascent of mouse: advances in modelling human depression and anxiety. Nat Rev Drug Discov. 4, 775-790 (2005).
(22) Ripoll, N., David, D., Dailly, E., Hascoet, M. & Bourin, M. Antidepressant-like effects in various mice strains in the tail suspension. Behav Brain Res. 143:193-200. 143, 193-200 (2003).
(23) Daugé, V., Sebret, A., Beslot, F., Matsui, T., & Roques B. Behavorial profile of CCK2 receptor-deficient mice. Neuropsychopharmacol. 25, 690-698 (2001).
(24) Santarelli, L., Saxe, M, Gross, C, Surget, A, Battaglia, F, Dulawa, S, Weisstaub, N, Lee, J, Duman, R, Arancio, O, Belzung, C, Hen, R et al, Requirement of hippocampal neurogenesis for the behavioral effects of antidepressants, Science, 301 : 805-809 (2003). '
(25) Heurteaux C., Guy, N.,Laigle, C.,Blondeau, N.,Duprat, F.,Mazzuca, M.,Lang-Lazdunski, L.,Widmann, C. et al, TREK-1, a K(+) channel involved in neuroprotection and general anesthesia, EMBO J, 23: 2684-2695 (2004).
(26) Tsirka S. E., Gualandris, A., Amaral, D. G., Strickland, S. Excitotoxin-induced neuronal degeneration and seizure are mediated by tissue plasminogen activator, Nature,377 : 340-344 (1995).
(27) Heurteaux C, Laigle, C., Blondeau, N., Jarretou, G., Lazdunski, M. Alpha-linolenic acid and riluzole treatment confer cerebral protection and improve survival after focal brain ischemia, Neuroscience, 137 : 241-251 (2006).
(28) Sheline, Y. I.,Wang, P. W., Gado, M. H., Csernansky, J. G.,Vannier, M. W. Hippocampal atrophy in recurrent major depression, Proc Natl Acad Sci U S A. 93 3908-3913 (1996)
(29) Gould, E., Tanapat, P., McEwen, B. S.,Flugge, G., Fuchs, E. Proliferation of granule cell precursors in the dentate gyrus of adult monkeys is diminished by stress, Proc Natl Acad Sci U S A, 95: 3168-3171 (1998).

## Revendications

1. Peptide de séquence ID N°2.

2. Séquence d'acide nucléique codant un peptide selon la revendication 1.

3. Vecteur comprenant une séquence d'acide nucléique selon la revendication 2.

4. Cellule hôte comprenant un peptide selon la revendication 1 et/ou une séquence d'acide nucléique selon la revendication 2 et/ou un vecteur selon la revendication 3.

5. Procédé de production d'un peptide selon la revendication 1 comprenant les étapes suivantes :
- transfecter une cellule hôte avec un acide nucléique selon la revendication 2 ou transformer une cellule hôte avec un vecteur selon la revendication 3 ;
- cultiver ladite cellule hôte dans des conditions permettant l'expression du peptide selon la revendication 1 ; et
- récupérer ledit peptide ou fragment ou dérivé de ce peptide.

6. Utilisation d'un peptide selon la revendication 1 pour la fabrication d'un médicament destiné au traitement de troubles psychiatriques, de phénomènes inflammatoires et/ou douloureux.

7. Utilisation selon la revendication 6, pour la fabrication d'un antidépresseur.

## Claims

1. A peptide of sequence ID No. 2.

2. A nucleic acid sequence encoding a peptide according to claim 1.

3. A vector comprising a nucleic acid sequence according to claim 2.

4. A host cell comprising a peptide according to claim 1 and/or a nucleic acid sequence according to claim 2 and/or a vector according to claim 3.

5. A method of production of a peptide according to claim 1 comprising the following stages:
- transfecting a host cell with a nucleic acid according to claim 2 or transforming a host cell with a vector according to claim 3;
- cultivating said host cell in conditions permitting the expression of the peptide according to claim 1; and
- recovering said peptide or fragment or derivative of said peptide.

6. A use of a peptide according to claim 1 for the manufacture of a medicinal product intended for the treatment of psychiatric disorders, and of inflammatory and/or painful phenomena.

7. The use according to claim 6, for the manufacture of an antidepressant.

## Patentansprüche

1. Peptid mit der Sequenz ID Nr. 2.

2. Nukleinsäuresequenz, die für ein Peptid nach Anspruch 1 kodiert.

3. Vektor, der eine Nukleinsäuresequenz nach Anspruch 2 umfasst.

4. Wirtszelle, die ein Peptid nach Anspruch 1 und/oder eine Nukleinsäuresequenz nach Anspruch 2 und/oder einen Vektor nach Anspruch 3 umfasst.

5. Verfahren zur Herstellung eines Peptids nach Anspruch 1, das folgende Schritte umfasst:
- Transfektion einer Wirtszelle mit einer Nukleinsäure nach Anspruch 2 oder Umwandeln einer Wirtszelle mit einem Vektor nach Anspruch 3;
- Kultivieren der besagten Wirtszelle unter Bedingungen, welche die Expression des Peptids nach Anspruch 1 ermöglichen; und
- Wiedergewinnung des besagten Peptids oder eines Fragments oder eines Derivats dieses Peptids.

6. Verwendung eines Peptids nach Anspruch 1 zur Herstellung eines Medikaments zur Behandlung von psychiatrischen Störungen und entzündlichen und/oder schmerzlichen Erscheinungen.

7. Verwendung nach Anspruch 6 zur Herstellung eines Antidepressivums.


### SEQUENCE LISTING

<110> centre National de la Recherche scientifique (CNRS)
   Université de Nice Sophia-Antipolis
   MAZELLA, Jean
   HEURTEAUX, Catherine
   BORSOTTO, Marc
   PETRAULT, Olivier
   WIDMANN, Catherine
<120> PEPTIDE DERIVE DE NSTR3 ET UTILISATION DANS LE TRAITEMEMENT DE
   MALADIES PSYCHIATRIQUES
<130> 103065/EP
<150> EP 07291602.6
   <151> 2007-12-21
<160> 2
<170> Patentin version 3.3
<210> 1
   <211> 44
   <212> PRT
   <213> Homo sapiens
<400> 1
   Gln Asp Arg Leu Asp Ala Pro Pro Pro Pro Ala Ala Pro Leu Pro Arg
   1               5                   10                  15
   Trp Ser Gly Pro Ile Gly Val Ser Trp Gly Leu Arg Ala Ala Ala Ala
               20                  25                  30
   Gly Gly Ala Phe Pro Arg Gly Gly Arg Trp Arg Arg
           35                  40
<210> 2
   <211> 17
   <212> PRT
   <213> Homo sapiens
<400> 2
   Ala Pro Leu Pro Arg Trp Ser Gly Pro Ile Gly Val Ser Trp Gly Leu
   1               5                   10                  15
   Arg